# EUROPÄISCHE PATENTANMELDUNG

(11) **EP 0 867 163 A2**
(43) Veröffentlichungstag der Anmeldung: **30.09.1998**
(21) Anmeldenummer: 98100930.1
(22) Anmeldetag: 21.01.1998
(51) Int. Cl.: A61F 13/15, A61F 13/56

(54) **Verschlussband für eine Windel und Verfahren zum Verschliessen einer derartigen Windel**

(30) Priorität: 26.03.1997 DE 19712601; 12.08.1997 DE 29714334 U
(71) Anmelder: Koester GmbH & Co. KG, 96146 Altendorf (DE)
(72) Erfinder: Bräunig, Werner, 97514 Oberaurach (DE); Schober, Uwe, 96179 Rattelsdorf (DE)
(74) Vertreter: Schneck, Herbert, Dipl.-Phys., Dr.

(57) **Zusammenfassung**

Bei einem Verschlußband für eine Windel umfassend einen mit der Windel fest zuverbindenden ersten Haftabschnitt, einen elastischen Zwischenabschnitt und einen hieran anschließenden zweiten Haftabschnitt zum Verschließen der Windel, wobei zwei derartige Verschlußbandabschnitte zu beiden Seiten einer Windel angeordnet werden, ist zur Erzielung einer leichten Verschließbarkeit der Windel im gebrauchten Zustand vorgesehen, daß der elastische Zwischenabschnitt (5) so ausgestaltet ist, daß er relativ zu dem an einer Windel (1) angebrachten zweiten elastischen Zwischenabschnitt des zweiten Verschlußbandabschnitts (3) ein gutes Haftvermögen, andererseits aber relativ zur Windelaußenseite kein oder nur ein geringes Haftvermögen aufweist.

Ein Verschließen der gebrauchten Windel kann auch dadurch bewerkstelligt werden, daß die Verschlußbänder im Bereich ihrer elastischen Zwischenabschnitte miteinander verdrillt werden.

## Beschreibung

Die Erfindung richtet sich auf ein Verschlußband für eine Windel umfassend einen mit der Windel fest zu verbindenden ersten Haftabschnitt, einen elastischen Zwischenabschnitt und einen hieran anschließenden zweiten Haftabschnitt zum Verschließen mit der Windel, wobei zwei derartige Verschlußbandabschnitte zu beiden Seiten einer Windel angeordnet werden.

Soweit im Vorstehenden von einem Haftabschnitt die Rede ist, ist dieser insbesondere durch ein mechanisches Verschlußsystem, wie eine Haken- und Ösenanordnung, gebildet sein.

Bei Wegwerf-Windeln der in Betracht stehenden Art besteht das Problem, daß die gebrauchte, Exkremente enthaltende Windel häufig noch für längere Zeit in einen, häuslichen Abfallbehälter aufbewahrt werden muß, so daß es wichtig ist, daß diese gebrauchte Windel nach außenhin so dicht abgeschlossen ist, daß weder der eingeschlossene Inhalt noch Geräche nach außen dringen können.

Um einen derartigen Verschluß zu ermöglichen, gibt es zahlreiche Lösungsansätze, welche zusätzliche Verschlußbänder oder zusätzliche Haftabschnitte an den für den Tragzustand vorgesehenen Verschlußbändern einerseits und andererseits besonders ausgebildetete Klebe- und Haftzonen an der Windeloberfläche umfassen. Sämtliche vorbekannte Konstruktionen haben den Nachteil, daß sie vergleichsweise aufwendig sind und den Aufbau des Verschlußbandes komplizieren.

Hiervon ausgehend liegt der Erfindung die Aufgabe zugrunde, ein Verschlußband der eingangs genannten Art so auszugestalten, daß einerseits ein zuverlässiger, einfach realisierbarer Verschluß der gebrauchten Windel ermöglicht wird, andererseits aber kostenaufwendige Zusatzmaßnahmen vermieden werden.

Diese Aufgabe wird erfindungsgemäß dadurch gelöst, daß der elastische Zwischenabschnitt so ausgestaltet ist, daß er relativ zu den, an der Windel angebrachten zweiten elastischen Zwischenabschnitt ein gutes Haftvermögen aufweist, andererseits aber relativ zur Windelaußenseite kein oder nur ein geringes Haftvermögen.

Diese Haftfähigkeit der elastischen Abschnitte relativ zueinander wird vorzugsweise dadurch erreicht, daß polare Substanzen in das Elastomer eingearbeitet sind, wobei die Adhäsion aufgrund dieser Polarität zwischen zwei die gleiche Substanz enthaltenden elastischen Abschnitten groß und gegenüber der Windeloberfläche klein ist.

Günstigerweise werden polare Weichmacher in das Elastomer eingearbeitet.

Die Erfindung richtet sich auch auf ein Verfahren zum Verschließen einer gebrauchten Windel, welche zum Verschließen im Tragzustand mit wenigstens zwei Verschlußbändern, z. B. Klebebändern oder mechanischen Verschlußsystemen, versehen ist, die jeweils wenigstens einen elastischen Zwischenabschnitt aufweisen.

Ein derartiges Verfahren zeichnet sich dadurch aus, daß die Windel zu einem geschlossenen Bündel zusammengelegt wird derart, daß die elastischen Zwischenabschnitte der wenigstens zwei Klebebänder in Kontakt kommen, wobei die Zwischenabschnitte bzw. die Klebebänder dann gegeneinander verdrillt werden.

Es wurde festgestellt, daß durch ein derartiges Verdrillen eine außerordentlich feste und stabile Haftung erreicht wird, so daß ein hoher Zug beim Verschließen des Windelbündels aufgebracht und gehalten werden kann, was zu einem hermetischen Abschluß bei höchster Geruchsneutralität führt.

Unter "Verdrillen" ist im übrigen nicht nur ein exakt spiralförmiges Eindrehen der beiden Zwischenabschnitte zu verstehen, sondern auch ein Ineinanderdrehen, Verschlingen oder dergleichen der beiden Klebebänder im Bereich ihrer elastischen Zwischenabschnitte. Unter praktischen Erwägungen ist die mechanische Verbindung der beiden Bänder so vorzunehmen, daß die beiden Bänder nicht von selbst wieder aufgehen.

Zur Durchführung des Verfahrens sind die Klebebänder vorzugsweise so ausgebildet, daß sie zwei Trägerabschnitte aufweisen, die zumindest in ihrem Verbindungsbereich mit einem Zwischenabschnitt mit einer offenen Oberfläche versehen sind, und der Zwischenabschnitt aus thermoplastischem, elastischem Material besteht, wobei durch Eindringen des Materials im erweichten Zustand in die Oberflächenöffnungen der Trägerabschnitte der Zwischenabschnitt in den Trägerabschnitten mechanisch verankert ist. Derart ausgebildete Klebebänder weisen besonders vorteilhafte Eigenschaften hinsichtlich der Haltbarkeit in, verdrillten Zustand auf.

Die Verbindung zwischen den beiden Klebebändern kann vorzugsweise noch dadurch stabilisiert werden, daß das thermoplastische, elastische Material der Zwischenabschnitte eine adhäsive oder klebfähige Oberfläche aufweist.

Nachfolgend wird die Erfindung anhand bevorzugter Ausführungsbeispiele näher erläutert. Dabei zeigen:
- Fig. 1: eine schematische Aufsicht auf ein für die Durchführung des erfindungsgemäßen Verfahrens geeignetes Verschlußband,
- Fig. 2: einen schematischen Schnitt durch das Verschlußband längs der Linie II-II in Fig. 1,
- Fig. 3: eine Aufsicht auf ein erfindungsgemäß verschlossenes Windelpaket und
- Fig. 4: ein Ausführungsbeispiel für ein Windelverschlußband mit adhäsiven Abschnitten.

Ein in der Zeichnung dargestelltes Verschlußband V besteht aus einem ersten Trägerabschnitt 1 aus unelastischem thermofixierten Polypropylen-Vlies. Ein zweiter Trägerabschnitt 2 besteht aus dem gleichen Material, welches sich durch eine offene Oberfläche und von außen zugängliche Zwischenräume zwischen den Vliesfasern auszeichnet.

Beide rechteckig ausgebildete Trägerabschnitte 1, 2 sind durch einen streifenförmigen Zwischenabschnitt 3 miteinander verbunden. Der Zwischenabschnitt 3 besteht aus thermoplastischem, elastischem Material.

Bei der Herstellung wird auf zwei in Produktionsrichtung P im Abstand a voneinander laufende Vliesstreifen der Zwischenabschnitt 3 aufextrudiert. Das thermoplastische Material ist geschmolzen und kann in die Zwischenräume des Vliesmaterials eindringen. Durch das Verhaken des thermoplastischen Materials wird eine mechanische Verbindung zwischen dem Zwischenabschnitt 3 innerhalb der Verbindungsbereiche 4, 5 mit den Trägerabschnitten 1, 2 erreicht.

Auf die beiden Trägerabschnitte 1, 2 ist über eine Permanentkleberschicht 6 jeweils ein Folienstreifen 7 aufgebracht, mit dem die Trägerabschnitte 1, 2 verstärkt und mit einer glatten Oberfläche versehen werden. Auf dem Folienstreifen 7 des Trägerabschnitts 2 wird über eine wieder ablösbare Kleberschicht 8 ein Ablösestreifen 9 aufgebracht, der teilweise den Zwischenabschnitt 3 übergreift.

Der gesamte beschriebene Aufbau des Verschlußbandes V ist mit einer Permanentkleberschicht 10 abgedeckt, die direkt auf dem Folienstreifen 7 des Trägerabschnitts 1, der freiliegenden Oberfläche des Zwischenabschnitts 3 und dem Ablösestreifen 9 verläuft. Weiterhin ist auf der Permanentkleberschicht 10 noch ein zentraler Streifen 11 vorgesehen, der sich von dem Ablösestreifen 9 über den Zwischenabschnitt 3 zu dem Folienstreifen 7 erstreckt.

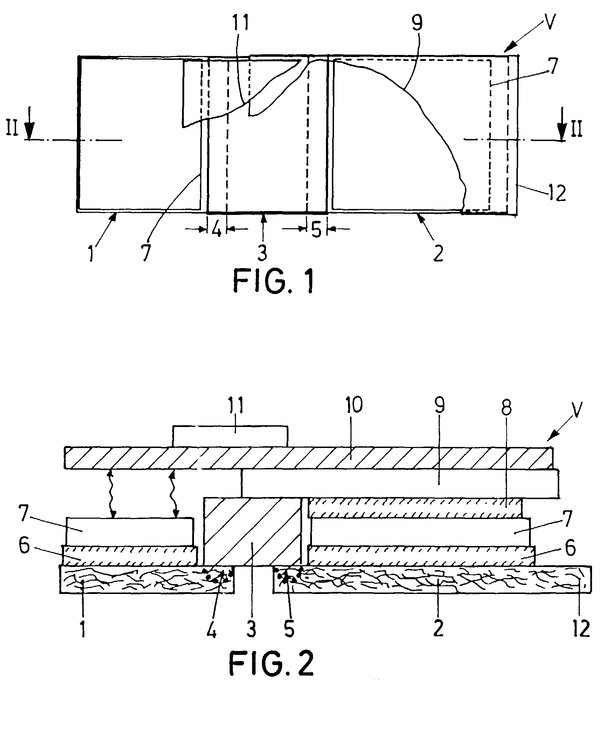

Wie aus Fig. 1 und 2 ferner deutlich wird, bildet der Trägerabschnitt 2 durch seinen seitlichen Überstand über die Permanentkleberschicht 6 eine Grifflasche 12, die bei der Anwendung der Windel erfaßt und damit der Trägerabschnitt 2 mit dein Folienstreifen 7 und der Kleberschicht 8 vom Ablösestreifen 9 abgezogen wird.

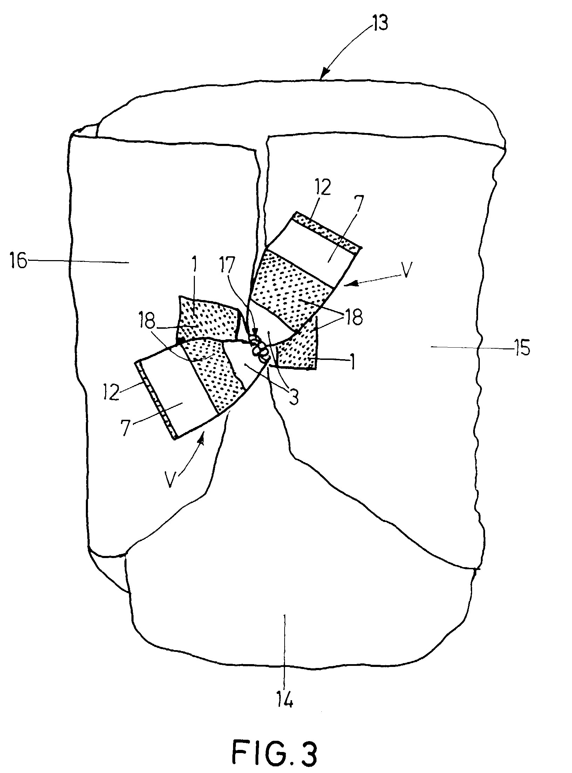

In Fig. 3 ist eine gebrauchte Baby-Windel 13 gezeigt, die mit Hilfe der in den Fig. 1 und 2 detailliert dargestellten Verschlußbänder V zu einem dicht abgeschlossenen Bündel fixiert ist. Dazu ist das zentrale Hauptteil 14 der Windel 13, das mit dem im gebrauchten Zustand verschmutzten Saugkörper der Windel versehen ist, auf sich gerollt. Die vom Rückenteil der Windel 13 abstehenden, im angelegten Zustand um die Hüften herumreichenden Flügel 15, 16 sind straff um das gerollte zentrale Hauptteil 14 herumgezogen. Die beiden Verschlußbänder V kommen dann obenauf zu liegen, wie dies in Fig. 3 erkennbar ist. Diese Verschlußbänder V sind jeweils mit ihren Trägerabschnitten 1 über die Permanentkleberschicht 6, den Verstärkungs-Folienstreifen 7 und den darunter befindlichen Teil der Permanentkleberschicht 10 mit dem jeweiligen Flügel 15, 16 der Windel 13 dauerhaft verklebt.

Wie in Fig. 3 angedeutet ist, werden zum dauerhaften Verschließen der gebündelten Windel 13 die beiden Verschlußbänder V zusammengeführt und mit ihren elastischen Zwischenabschnitten 3 in Kontakt gebracht. Durch Verdrillen dieser Zwischenabschnitte 3 - in Fig. 3 ist die Verdrillung 17 durch verschlungene Linien angedeutet - werden die Verschlußbänder V dauerhaft und unter hohem Zug auf die beiden Flügel 15, 16 miteinander verbunden. Durch die adhäsiven, leicht klebfähigen Oberflächeneigenschaften der Zwischenabschnitte 3 wird die mechanische Verbindung zwischen den Zwischenabschnitten 3 noch zusätzlich stabilisiert und unterstützt.

In Fig. 3 sind ferner die jeweiligen verstärkenden Folienstreifen 7 auf den Trägerabschnitten 2 und die Grifflaschen 12 erkennbar. Ferner ist durch eine Punktschraffur eine Prägung 18 der aus Vliesmaterial bestehenden Trägerabschnitte 1 und 2 angedeutet.

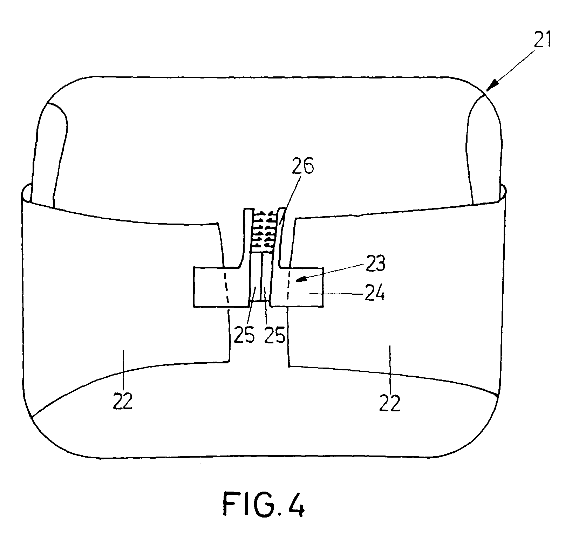

Die in Fig. 4 dargestellte Windel 21 umfaßt zwei Ansätze 22, an welchen in an sich bekannter Weise ein Verschlußband 23 befestigt ist, welches einen fest mit diesen Ansätzen 22 verbundenen Abschnitt 24, jeweils einen elastischen Zwischenabschnitt 25 und einen weiteren Haftabschnitt 26 aufweist, der zur Befestigung im normalen Tragzustand verwendet wird.

Zur Entsorgung der benutzten Windel werden die elastischen Zwischenabschnitt 25 (die in der Zeichnung um 90° verdreht dargestellt sind) gegeneinander gedrückt, wobei die aufgrund ihrer wechselseitigen Adhäsion in dieser Position verbleiben.

## Patentansprüche

1. Verschlußband für eine Windel umfassend einen mit der Windel fest zu verbindenden ersten Haftabschnitt, einen elastischen Zwischenabschnitt und einen hieran anschließenden zweiten Haftabschnitt zum Verschließen der Windel, wobei zwei derartige Verschlußbandabschnitte zu beiden Seiten einer Windel angeordnet werden, **dadurch gekennzeichnet, daß** der elastische Zwischenabschnitt (5) so ausgestaltet ist, daß er relativ zu dem an einer Windel (1) angebrachten zweiten elastischen Zwischenabschnitt des zweiten Verschlußbandabschnitts (3) ein gutes Haftvermögen, andererseits aber relativ zur Windelaußenseite kein oder nur ein geringes Haftvermögen aufweist.

2. Verschlußband nach Anspruch 1, **dadurch gekennzeichnet, daß** polare Substanzen in das Elastomer eingearbeitet sind.

3. Verschlußband nach Anspruch 2, **dadurch gekennzeichnet, daß** polare Weichmacher in das Elastomer eingearbeitet sind.

4. Verfahren zum Verschließen einer gebrauchten Babywindel (13), welche zum Verschließen im Tragzustand mit wenigstens zwei Klebe-Verschlußbändern (V) versehen ist, die jeweils wenigstens einen elastischen Zwischenabschnitt (3) aufweisen, **dadurch gekennzeichnet, daß** die Windel (13) zu einem geschlossenen Bündel zusammengelegt wird derart, daß die elastischen Zwischenabschnitte (3) der wenigstens zwei Klebe-Verschlußbänder (V) in Kontakt kommen.

5. Verfahren nach Anspruch 4, **dadurch gekennzeichnet, daß** die Zwischenabschnitt (3) gegeneinander verdrillt werden.

6. Verfahren nach Anspruch 4, **dadurch gekennzeichnet, daß** die Klebe-Verschlußbänder (V) zwei Trägerabschnitte (1, 2) aufweisen, die zumindest in ihrem Verbindungsbereich (4, 5) mit einem Zwischenabschnitt (3) mit einer offenen Oberfläche versehen sind und der Zwischenabschnitt (3) aus thermoplastischem, elastischen Material besteht, wobei durch Eindringen des Materials im erweichten Zustand in die Oberflächenöffnungen der Trägerabschnitte (1, 2) der Zwischenabschnitt (3) in den Trägerabschnitten (1, 2) mechanisch verankert ist.

7. Verfahren nach Anspruch 4, **dadurch gekennzeichnet, daß** die Zwischenabschnitte (3) der Klebe-Verschlußbänder (V) mit einer adhäsiven oder leicht klebfähigen Oberfläche versehen sind.
